# EUROPÄISCHE PATENTSCHRIFT

(11) **EP 2 941 412 B1**
(45) Veröffentlichungstag und Bekanntmachung des Hinweises auf die Patenterteilung: **24.10.2018**
(21) Anmeldenummer: 13829059.8
(22) Anmeldetag: 20.12.2013
(51) Int. Cl.: B01D 1/00, B01D 15/02, B01D 61/02, B01D 9/00, C07C 51/44, C07C 51/47, C07C 51/43

(54) **VERFAHREN ZUR AUFREINIGUNG VON CARBONSÄUREN AUS FERMENTATIONSBRÜHEN**
METHOD FOR PURIFYING CARBOXYLIC ACIDS FROM FERMENTATION BROTHS
PROCÉDÉ DE PURIFICATION D'ACIDES CARBOXYLIQUES ISSUS DE BOUILLONS DE FERMENTATION

(30) Priorität: 03.01.2013 DE 102013000027
(43) Veröffentlichungstag der Anmeldung: 11.11.2015
(73) Patentinhaber: thyssenkrupp Industrial Solutions AG, 45143 Essen (DE)
(72) Erfinder: KLEIBER, Michael, 65795 Hattersheim (DE); GNABS, Ulrike, 65779 Kelkheim (DE); SCHULZE, Joachim, 59494 Soest (DE); GHANEGAONKAR, Shashank, 04109 Leipzig (DE); GEHRKE, Helmut, 59192 Bergkamen (DE); GAWENDA, Marcel, 46238 Bottrop (DE)
(74) Vertreter: thyssenkrupp Intellectual Property GmbH
(86) Internationale Anmeldenummer: PCT/EP2013/003892
(87) Internationale Veröffentlichungsnummer: WO 2014/106532

(56) Entgegenhaltungen:
- WO-A2-2011/082378
- DE-A1-102010 025 167

## Beschreibung

Die Erfindung betrifft ein Verfahren zur Aufreinigung von Carbonsäuren, ausgewählt aus der Gruppe umfassend Hydroxycarbonsäuren und Dicarbonsäuren, aus Fermentationsbrühen, sowie die Verwendung einer Vorrichtung zur Durchführung des erfindungsgemäßen Verfahrens. Die Isolierung von Carbonsäuren, die nicht oder nur schwer durch Destillation abgetrennt werden können, gestaltet sich sehr aufwendig.

Entscheidend für die industrielle Nutzung von Carbonsäuren, die durch Fermentation kohlehydrathaltiger Substrate mittels verschiedener Mikroorganismen erzeugt werden, ist die Wirtschaftlichkeit und Effizienz der Abtrennung und Reinigung der Milchsäure aus diesen wässrigen Fermentationslösungen, die neben der Carbonsäure oder den Carbonsäuresalzen auch weitere organische Säuren, sonstige Nebenprodukte der Fermentation, Mikroorganismen und deren Bestandteile sowie Reste der Substrate, wie Zucker, enthalten. Diese Verunreinigungen stören bei der anschließenden Weiterverarbeitung der erzeugten Carbonsäuren. Beispielsweise wird Milchsäure zu Polymilchsäure polymerisiert, um biologisch abbaubare Kunststoffe herzustellen. Hierzu muss, um einen hohen Polymerisationsgrad der Milchsäure zu erreichen, extrem reines Monomer eingesetzt werden. Dies ist seit langem bekannt und geht beispielsweise aus J. Dahlmann et al, British Polymer Journal, Bd. 23 (1990), S. 235. 240 hervor.

Ähnliches ist beispielsweise für Bernsteinsäure bekannt. Unterschieden werden können die Qualitäten der erzeugten Bernsteinsäure durch die Unterteilung in eine technische Qualität mit einem Bernsteinsäuregehalt von mindestens 97 Ma-% und einer speziell für die Verwendung zur Polymerisation geeigneten Bernsteinsäure (polymer grade) mit einem Gehalt von mindestens 99,5 Ma-%.

Eine Vielzahl von Patenten beschreibt die Gewinnung von Bernsteinsäure aus Fermentationslösungen, darunter
- extraktive Prozesse unter Verwendung von Extraktionsmitteln wie Tributylaminen, Trialkylaminen, Olefinen, verschiedenen Alkoholen und aromatischen Kohlenwasserstoffen,
- Prozesse unter Verwendung von Calciumhydroxid und Schwefelsäure, wobei als Nebenprodukt Gips anfällt,
- Prozesse unter Verwendung der Elektrodialyse,
- Thermische Methoden wie fraktionierte Destillation oder thermisch gestufte Chromatographie,
- Hochdruckextraktion unter Verwendung von CO₂,
- Membranverfahren wie beispielsweise Umkehrosmose und sonstige Filtrationsprozesse
wobei auch Kopplungen dieser Verfahren und Ergänzung durch weitere dem Stand der Technik entsprechende Schritte diskutiert werden. Derartige Verfahren werden unter anderem in den Patentschriften DE 69821951 T2; DE 69015233 T2 ; DE 69015019 T2; DE 69006555 T2 ; DE 69015019 ; DE19939630C2; DE 60028958T2 ; DE 10 2004 026152 A1 beschrieben.

Weiterhin sind eine Vielzahl von Methoden betreffend die Aufreinigung von Milchsäure bekannt.

Beispielsweise wird in einigen Patenten gelehrt, die Destillation zur Aufreinigung von Milchsäure aus wässrigen Lösungen zu nutzen. Ein derartiges Verfahren macht sich die EP 0986532 B2 zu nutze. In der DE 10 2007 045 701 B3 wird eine kombinierte Extraktion mit linearem n-Trioctylamin (TOA) und einer Destillation offenbart. Weitere in der Literatur bekannte Möglichkeiten sind die Elektrodialyse bzw. die Veresterung mit einem Alkohol, wonach ebenfalls eine Destillation und dann eine Hydrolyse des gebildeten Esters durchgeführt werden. Diese Verfahren sind äußerst Kosten-intensiv. Die Destillation bietet zudem den Nachteil, dass immer auch ein Teil der Kohlenhydrate mitextrahiert werden, was zu einer Verschlechterung der Ausbeute des gesamten Prozesses führt und die Isolierung des Produktes erschwert.

Auch Prozesse unter der Verwendung von Calciumhydroxid und Schwefelsäure, wobei als Nebenprodukt Gips in großen Mengen anfällt, sind bekannt. In diesem Zusammenhang wurde zudem gefunden, dass sich Milchsäure aus beispielsweise einer mit Schwefelsäure angesäuerten Fermentationsbrühe, die neben freier Milchsäure noch Ammonium- und Sulfationen enthält, mittels chromatographischer Methoden isolieren lässt. DE 69815369 T2 beschreibt beispielsweise unter anderem die Abtrennung von Milchsäure aus wässrigen Mischungen durch Adsorption an ein festes Adsorbens, vorzugsweise wird hier ein Feststoffadsorbens verwendet, das Milchsäure versus Lactat adsorbiert. Insbesondere kommen laut obiger Schrift schwache Anionenaustauscher zur Milchsäureisolierung in Frage. Die DE 10 2009 019 248 A1 beschreibt weiterhin chromatographische Methoden zur Aufreinigung organischer Säuren, im Speziellen von Milchsäure, indem eine Simulated Moving Bed Chromatographie durchgeführt wird.

Die WO 2006/124633 A1 beschreibt einen Prozess zur Herstellung von Ammoniumlactat durch Fermentation. Bei der Fermentation bildet sich das Ammoniumsalz der Milchsäure, das aus der Fermentationslösung z.B. durch Extraktion abgetrennt werden kann. Das Ammoniumsalz kann in einem Folgeschritt sehr leicht mit schwachen Säuren oder Kohlendioxid gespalten werden. Dabei gewinnt man die freie Milchsäure, die dann beispielsweise durch Destillation gereinigt werden kann.

Die WO99/19290 beschreibt eine Milchsäurefermentation mit anschließender Filtration und Extraktion, wobei die Extraktion eine Adsorption sein kann. Dabei ist die Art der Interaktion mit der festen Phase der Adsorption nicht offenbart. Ein ähnliches Verfahren wird in der WO93/06226 offenbart, wobei hier die feste Phase der Adsorption mit tertiären Aminogruppen ausgestattet ist und dadurch die Produktionsrate an freier Säure erhöht wird. Auch die EP0135728 lehrt die Isolierung von enzymatisch erzeugten Carbonsäuren über Adsorber, die mit tertiären Aminogruppen ausgestattet sind. Dabei erfolgt die Fermentation über an Säulen immobilisierte Zellen.

Die DE102010025167A1 offenbart ein Verfahren zur Abtrennung, Gewinnung und Reinigung von Bernsteinsäure. Dabei wird eine Abtrennung der Biomasse aus der Fermentationsbrühe in zwei aufeinanderfolgenden Verfahrensschritten erreicht. Anschließend erfolgt eine Abtrennung der Dicarbonsäurelösung aus der biomassefreien Fermentationsbrühe durch Simulated Moving Bed Chromatrographie, gefolgt von einer Feinreinigung und einer mehrstufigen Eindampfung und Kristallisation. Nachteilig an diesem Verfahren ist der hohe energetische Aufwand der durch die mehrstufige Eindampfung der Dicarbonsäurelösung hervorgerufen wird.

Die WO2011082378A2 lehrt die Reinigung von Bernsteinsäure aus einer Fermentationsbrühe, die Ammoniumsuccinat enthält. Es werden Ionenaustauschersäulen verwendet, um das Ammoniumsuccinat von der Fermentationsbrühe zu trennen und Bernsteinsäure zu generieren. Dabei wird Ammoniumsulfat im Raffinat und Bernsteinsäure im Extrakt erhalten. Das Raffinat wird einer Kristallisation unterzogen, wozu es aufkonzentriert wird. Die Aufkonzentrierung des Raffinats, also des Ammoniumsulfates, erfolgt über Umkehrosmose und/oder Eindampfung. Dieser Schritt ist allgemein bekannt. Die Aufbereitung des Ammoniumsulfats ist essentiell für die Wirtschaftlichkeit der fermentativen Herstellung von Bernsteinsäure, da bei der Umsetzung von Ammoniumsuccinat aus der Fermentationsbrühe zur Bernsteinsäure in etwa anderthalbfacher Menge Ammoniumsulfat anfällt.

Nachteil vieler Verfahren ist es also, dass die praktische Durchführung der Verfahren mit einem erheblichen technischen und energetischen Aufwand verbunden ist.

Aufgabe der Erfindung ist es, einen energetisch günstigen Gesamtprozess zur Abtrennung und Aufreinigung von Carbonsäuren aus Fermentationsbrühen zur Verfügung zu stellen, wobei bekannte Nachteile anderer Verfahren vermieden werden sollen.

Erfindungsgemäß wird die Aufgabe gelöst, durch den Einsatz eines Verfahrens zur Abtrennung und Aufreinigung von Carbonsäuren aus Fermentationsbrühen, wobei das Verfahren die folgenden Schritte umfasst,
a. eine Abtrennung von Biomasse und eventuell vorhandener Feststoffe aus der Fermentationsbrühe,
b. Herstellung einer Lösung enthaltend die gewünschte Carbonsäure und einer weiteren Lösung enthaltend Ammoniumsalze, indem die biomassefreie Fermentationsbrühe mit konzentrierter Schwefelsäure angesäuert und einer Simulated Moving Bed Chromatographie (SMB) unterzogen wird ,
c. eine Feinreinigung der Lösung enthaltend die gewünschte Carbonsäure aus Verfahrensschritt b),
d. eine Aufkonzentrierung der aufgereinigten Carbonsäurelösung aus Verfahrensschritt c),
e. Kristallisation der aufkonzentrierten Carbonsäurelösung aus Verfahrensschritt d)
f. eine Aufkonzentrierung der weiteren Lösung enthaltend Ammoniumsalze aus Verfahrensschritt b),
**dadurch gekennzeichnet, dass**
in Verfahrensschritt d) und f) bei der Aufkonzentrierung eine Kombination aus Umkehrosmose und Eindampfung durchgeführt wird und der Dampf aus der Eindampfung des Verfahrensschrittes f) in die Eindampfung aus Verfahrensschritt d) geleitet wird.

Die Umsetzung und Rückgewinnung der Ammoniumsalze ist essentiell für die Wirtschaftlichkeit der fermentativen Herstellung von Bernsteinsäure.

In vorteilhafter Ausgestaltung des Verfahrens wird Verfahrenschritt f) derart ausgelegt, dass in Verfahrensschritt d) im Normalbetrieb keinerlei Frischdampf zuzuführen ist oder 1 bis 10 t Frischdampf pro t Produktcarbonsäure zuzuführen ist, besonders bevorzugt 1,5 bis 4 t Frischdampf pro t Produktcarbonsäure zuzuführen ist. Normalbetrieb meint dabei den Betrieb nach dem Hochfahren der Anlage und beinhaltet keinerlei Störungen im Betriebsablauf.

In weiterer Ausführung erfolgt die Abtrennung der Biomasse aus der Fermentationsbrühe in Verfahrensschritt a) durch eine Precoat- und/oder eine Mikrofiltration und/oder eine Ultrafiltration und die abgetrennte Biomasse in Verfahrensschritt a) wird wieder in den Fermenter rückgeführt. Dabei entsprechen Temperatur und pH-Wert den Werten der Fermentation, da festgestellt wurde, dass durch Inaktivierung der Biomasse durch Temperaturerhöhung und Absenken des pH-Wertes durch Zugabe von Säure eine Autolyse der Biomasse beschleunigt wird und mehr Lyseprodukte in die Fermentationsbrühe abgegeben werden. Auch muss die Zeit zwischen Beendigung der Fermentation und der Abtrennung der Biomasse so kurz als möglich gehalten werden und nicht mehr als 2 h betragen, und vorzugsweise weniger als 1 - 2 h betragen. Die Biomassekonzentration im Filtrat sollte 1 g/l nicht übersteigen. Durch diese Prozessführung wird die Endproduktqualität positiv beeinflusst.

Für die Fermentation selbst können eine Vielzahl von Mikroorganismen eingesetzt werden, einschließlich Bakterien, Hefen und Pilze. Die Fermentationsbrühe kann auch verschiedene Recycleströme aus dem Gesamtverfahren enthalten.

Das Filtrat aus der Precoat- oder Mikrofiltration wird optional auch einer ein- oder zweistufigen Ultrafiltration zugeführt. Hier werden restliche Biomasseanteile, unlösliche Feststoffe und höhermolekulare Verbindungen abgetrennt. Als Optimum zwischen Produktqualität und Fluxraten der Membranen wurden Membranen mit einer Tenngrenze von ≤10 kDa ermittelt. Die Temperatur der flüssigen Medien sollte wegen des Löslichkeitskoeffizienten von Ammoniumsuccinat in Wasser ≥ 30 °C betragen. Das Retentat wird zur Precoat- oder Mikrofiltration zurückgeführt oder alternativ als Ausgangsprodukt für die Erzeugung von Dicarbonsäure technischer Qualität gesammelt bzw. verwendet und das Permeat wird der weiteren Behandlung zugeführt.

Im Permeat des Verfahrensschrittes a) liegt die Dicarbonsäure in Form ihres Salzes - im Falle von Bernsteinsäure beispielsweise in Form von Ammoniumsuccinat - vor. Zur Überführung in die Carbonsäure erfolgt die Zugabe und Einmischung von konzentrierter Schwefelsäure und damit verbunden eine Absenkung des pH-Wertes der Lösung auf Werte zwischen 2,2 bis 2,4. Dabei entsteht in stöchiometrischem Verhältnis Ammoniumsulfat. Zur Vermeidung von unerwünschter Ausfällung erfolgt dieser Prozessschritt bei Temperaturen zwischen 30°C bis 60°C und vorzugsweise in einem Bereich zwischen 30°C bis 40°C. Diese vorgereinigte Lösung steht für die Abtrennung und Reinigung der Dicarbonsäure zur Verfügung. Optional erfolgt die Ansäuerung auch zweistufig, indem die erste Ansäuerung vor dem Verfahrensschritt a) und die zweite Ansäuerung nach dem Verfahrensschritt a) erfolgt.

Die Trennung des sauren Permeates der Ultrafiltration erfolgt in einer Simulated Moving Bed Chromatographie. Diese stellt eine besonders leistungsfähige Variante der High Performance Liquid Chromatographie dar, wobei durch die Aufeinanderfolge von mehreren über Ventile miteinander verbundenen Trennsäulen in einer Endlosschleife eine große Anzahl theoretischer Böden verwirklicht und die Trennschärfe der Chromatographie erheblich verbessert wird. Als stationäre Phase kommen Kationenaustauscher und Anionenaustauscher zum Einsatz. Nach Aufgabe der Lösung wird die Dicarbonsäure an die stationäre Phase gebunden und nach mehrfacher Ausspülung der nicht gewünschten Anteile der Lösung aus dem System eluiert und als Extrakt gesondert abgeführt. Als Eluent kommen entmineralisiertes Wasser und/oder Brüdenkondensat zur Verwendung. Auch ist es möglich das Permeat aus der Umkehrosmose als Eluent einzusetzen. Durch die Rezirkulation des Wassers kann die Wirtschaftlichkeit des Gesamtverfahrens weiter gesteigert werden.

Es konnte gezeigt werden, dass im Extrakt mehr als 95 % der im Permeat der Ultrafiltration enthaltenen Dicarbonsäure gewonnen werden kann, wobei das Verhältnis zwischen Permeat der Ultrafiltration und Eluent im Bereich zwischen 1 : 1,5 und 1 : 2,5 variiert und acht in einer Endlosschleife geschalteten Anionenaustauscher-Säulen Verwendung fanden. Der Extrakt enthält nur noch geringe Mengen an Ammoniumsulfat, Essigsäure und Farbstoffe aus der Fermentorbrühe. Das ausgespülte Raffinat enthält maximal 1 g/l Dicarbonsäure sowie das Ammoniumsulfat, Begleitsalze aus der Fermentation wie Phosphate, Nitrate und Chloride.

Bei der Durchführung der SMB des Verfahrensschrittes b) werden die folgenden Bedingungen eingehalten:
- die Zugabe des Permeates aus der Filtration aus Verfahrensschritt a) und eines Eluents erfolgt kontinuierlich in einem Verhältnis Permeat: Eluent von 1 : 1,5 bis 1 : 2,5,
- die Carbonsäure bindet an eine stationäre Phase der SMB, wobei diese aus einem Kationenaustauscher und/oder einem Anionenaustauscher aufgebaut ist,
- der die Carbonsäure enthaltende Extrakt und das Raffinat, aufweisend einen Gehalt an Dicarbonsäure von ≤ 1 g/l, getrennt voneinander gesammelt werden,
- der Wirkungsgrad der Gewinnung von Carbonsäure aus dem Permeat der Filtration aus Verfahrensschritt a) beträgt ≥ 95%.

Für die Herstellung einer hochreinen Dicarbonsäure (polymer grade) erfolgt eine Feinreinigung des Extraktes aus der Simulated Moving Bed Chromatographie, wobei die Membranen eine Trenngröße von 100 bis 400 Da aufweisen. Es konnte gezeigt werden, dass eine Nanofiltration mit einer Trenngrenze um 200 Da gute Qualitätsergebnisse ergibt. Dabei wird der Prozess so geführt, dass das Retentat der Nanofiltration nicht mehr als 10% des Gesamtdurchsatzes beträgt. Das Retentat enthält neben der Carbonsäure noch Essigsäure sowie Farbstoffe und kann dem Ausgangsprodukt für die Erzeugung von Carbonsäure in technischer Qualität zugegeben werden.

In Abhängigkeit der Qualität der für die Fermentation eingesetzten Rohstoffe und der Prozessführung in der Fermentation wird aufgrund noch vorhandener Reste von Farbstoffen und Begleitstoffen eine zusätzliche Feinreinigung des Permeates aus der vorgeschalteten Nanofiltration oder des Extraktes aus der SMB-Chromatographie durchgeführt. In diesem Fall wird eine Feinreinigung durch Aktivkohlefiltration und/oder Ionenaustauscher. nachgeschaltet. Als lonenaustauscherharze kommen in Abhängigkeit von der chemischen Analyse der Verunreinigungen Kationen- und/oder Anionenaustauscher in Betracht.

In weiterer Ausgestaltung des erfindungsgemäßen Verfahrens werden in Verfahrensschritt d) mindestens eine oder mehrere Stufen zur Umkehrosmose mit mindestens einer oder mehreren Verfahrensschritten zur Eindampfung kombiniert.

Die Darstellung des Produktes Carbonsäure - sowohl in technischer als auch in polymer grade Qualität - erfolgt durch Kristallisation. Dabei wurde festgestellt, dass die Parameter der Prozessführung erheblichen Einfluss auf die Produktqualität haben.

Für die Erzielung einer technischen Qualität mit einem Carbonsäuregehalt von ≥ 97 Ma-% ist es ausreichend, wenn Verfahrensschritt d) und e) in einem einmaligen Durchlauf durch diese Verfahrensschritte erfolgen. Die Umkehrosmose und Eindampfung erfolgt bis zu einer Konzentration von 30 bis 50 Ma.-%. Als wesentlicher Parameter für die Produktqualität wurde der während der Kristallisation anzuwendende Temperaturgradient für die Kühlung der Lösung gefunden. Die Abkühlung sollte demnach in Schritten von 3 - 8 °C/min erfolgen und bevorzugt in Schritten von 3 - 5°C/min. Die erzeugten Kristalle werden dann von der Mutterlauge durch Separation abgetrennt, mit Warmwasser von 40 °C gewaschen und die Mutterlauge vor die Eindampfung zurückgeführt. Die Kristalle werden nach der Separation getrocknet.

Für die Erzielung einer polymer grade- Qualität der Carbonsäure mit einem Gehalt von ≥ 99,5 Ma-% wurde gefunden, dass dabei die Temperatur im Bereich von 70 °C bis 80 °C liegen sollte und die Lösung auf eine Konzentration von 50 ± 5 Ma-% eingestellt werden muss. Als wesentlich für die Qualität der Kristalle wurde der Temperaturgradient während der Kühlung der Lösung festgestellt. Demnach erfolgt die Abkühlung in Schritten von 1 °C bis 5 °C/h. Damit werden Kristalle in polymer grade - Qualität erzeugt, die durch Separation abgetrennt und getrocknet werden. Die Mutterlauge kann zurückgeführt werden. Erforderlichenfalls kann nach der Separation eine Auflösung der Kristalle mit entmineralisiertem Wasser und/oder Brüdenkondensat erfolgen und der Schritt der Kristallisation und Separation wiederholt werden.

Vorteilhaft werden die Kristalle der Carbonsäure nach der Kristallisation durch Separation abgetrennt, wobei eine anfallende Mutterlauge vor die Eindampfung zurückgeführt wird und anschließend eine Trocknung der Kristalle erfolgt.

Um das Verfahren wirtschaftlich effektiv zu gestalten werden in Verfahrensschritt f) die Ammoniumsalze aus Verfahrensschritt c) aufkonzentriert.

Optional werden die Retentate der verschiedenen Filtrationen aus Verfahrensschritt c) zusammengeführt und als Ausgangslösung für die Herstellung einer Carbonsäure in technischer Qualität (technical grade) dienen. Die getrockneten Kristalle werden für die weitere Verwendung konfektioniert.

Das erfindungsgemäße Verfahren dient zur Reinigung von Carbonsäuren, ausgewählt aus der Gruppe umfassend Hydroxycarbonsäuren und Dicarbonsäuren, und die bevorzugt ausgewählt werden aus der Gruppe umfassend Äpfelsäure, Glycolsäure, Isozitronensäure, Mandelsäure, Milchsäure, Tartronsäure, Weinsäure, Zitronensäure, ß-Hydroxybuttersäure, Mevalonsäure, Salicylsäure, Oxalsäure, Maleinsäure, Bernsteinsäure, Glutarsäure, Adipinsäure, Pimelinsäure, Korksäure, Fumarsäure und Itaconsäure.

Die vorliegende Erfindung umfasst zudem die Verwendung einer Vorrichtung zur Durchführung eines Verfahrens nach Anspruch 1 umfassend
a) eine oder mehrere Filtrationseinrichtungen zur Abtrennung der Biomasse und eventuell vorhandenen Feststoffen aus der Fermentationsbrühe,
b) einen Verfahrensschritt zur Simulated Moving Bed Chromatographie (SMB) nach Ansäuerung, wobei eine Lösung enthaltend die gewünschte Carbonsäure und eine weitere Lösung enthaltend Ammoniumsalze entsteht,
c) einen Verfahrensschritt zur Feinreinigung der Lösung enthaltend die gewünschte Carbonsäure aus Verfahrensschritt b)
d) einen Verfahrensschritt zur Aufkonzentrierung der aufgereinigten Carbonsäurelösung aus Verfahrensschritt c)
e) einen Verfahrensschritt zur Kristallisation der aufkonzentrierten Carbonsäurelösung aus Verfahrensschritt d)
f) einen Verfahrensschritt zur Aufkonzentrierung der weiteren Lösung enthaltend Ammoniumsalze aus Verfahrensschritt b)
**dadurch gekennzeichnet, dass**
Verfahrensschritt d) und f) eine Kombination aus einer oder mehreren Vorrichtungen zur Umkehrosmose und einer oder mehreren Vorrichtungen zur Eindampfung aufweist, und Überleitungen vorhanden sind, um den Dampf aus der Eindampfung aus Verfahrensschritt f) in die Eindampfung aus Verfahrensschritt d) zu überführen.

In vorteilhafter Ausgestaltung der erfindungsgemäßen Verwendung einer Vorrichtung sind in Verfahrensschritt d) Anlagenteile zur Generierung von Frischdampf auf eine Frischdampfgenerierung von 1 bis 10 t Frischdampf pro t Produktcarbonsäure ausgelegt, und insbesondere auf eine Frischdampfgenerierung von 1,5 bis 4 t Frischdampf pro t Produktcarbonsäure ausgelegt.

Weiterin ist es vorteilhaft, wenn Überleitungen vorhanden sind, um Permeat aus der Umkehrosmose in die Simulated Moving Bed Chromatographie aus Verfahrensschritt b) zu überführen. Auf diese Weise kann das Gesamtverfahren noch wirtschaftlicher gestaltet werden.

### Beispiel 1

Eine Ammonuimsuccinat enthaltende Fermentorbrühe wurde entsprechend der Beschreibung durch Filtration vorgereinigt. Nach Überführung des Ammoniumsalzes in die Säureform der Bernsteinsäure wurde die Lösung in einer Simulated Moving Bed Chromatographie in 5,7 I Extrakt und 6,6 I Raffinat aufgetrennt. Dabei wurden insgesamt 8 Trennsäulen mit einem stark sauren Kationenaustauscher zu einer Endlosschleife verschaltet. Bei einem Permeat-/ Eluentverhältnis von 2,4 betrug der Koeffizient der Bernsteinsäuregewinnung 99,9 %. Die Sulfatkonzentration im Extrakt betrug 238 mg/l und im Raffinat 35.709 mg/l, womit eine Sulfatelimination von 99,4% erzielt wurde.

### Beispiel 2

Eine Ammonuimsuccinat enthaltende Fermentorbrühe wurde entsprechend der Beschreibung durch Filtration vorgereinigt. Nach Überführung des Ammoniumsalzes in die Säureform der Bernsteinsäure wurde die Lösung in einer Simulated Moving Bed Chromatographie in 5,3 I Extrakt und 6,1 I Raffinat aufgetrennt. Dabei wurden insgesamt 8 Trennsäulen mit einem stark sauren Kationenaustauscher zu einer Endlosschleife verschaltet. Bei einem Permeat-/ Eluentverhältnis von 2,2 betrug der Koeffizient der Bernsteinsäuregewinnung 99,8 %. Es wurde eine Sulfatelimination von 97,9% erzielt

### Beispiel 3

Ein die Bernsteinsäure enthaltender Extrakt aus der Simulated Moving Bed Chromatographie wurde einer Feinreinigung durch Nanofiltration mit einer Trenngrenze von 200 Da unterzogen. Im Extrakt wurden Gehalte von 44,8 g/l Bernsteinsäure und 698 mg/l Sulfate analysiert. Der filtrierte Extrakt wurde aufkonzentriert, kristallisiert und analysiert. Die Kristalle hatten einen Bernsteinsäuregehalt von 1.031 g/l und einen Gehalt an restlichen Sulfaten von 21,9 mg/l und Chloriden von 13,8 mg/l. Die Farbe der Kristalle war "weiß".

### Beispiel 4

Ein die Bernsteinsäure enthaltender Extrakt aus der Simulated Moving Bed Chromatographie mit einem Gehalt von 44,77 g/l Bernsteinsäure und 699 mg/l Sulfaten wurde einer Feinreinigung durch Nanofiltration mit einer Trenngrenze von 200 Da und folgender Aktivkohlefiltration unterzogen. Die nach der Feinreinigung erzeugten Kristalle wiesen einen Gehalt an Bernsteinsäure von 1.065 g/l und restlichen Sulfaten von 35,3 mg/l sowie von 9,5 mg/l Chloriden auf. Die Farbe der Kristalle war "reinweiß".

### Beispiel 5

Ein die Bernsteinsäure enthaltender Extrakt aus der Simulated Moving Bed Chromatographie wurde einer Feinreinigung mittels lonenaustausch unterzogen. Der Extrakt hatte einen Gehalt von 44,8 g/l Bernsteinsäure, 699 mg/l Sulfate und 1.88 mg/l Chloride. Die aus der feingereinigten Lösung erzeugten Kristalle wiesen einen Gehalt an Bernsteinsäure von 967 g/l Bernsteinsäure, 37,6 mg/l Sulfate und 0,92 mg/l Chloride auf. Die Farbe der Kristalle war "weiß".

### Beispiel 6

Analog Beispiel 2 erhaltenes Raffinat, welches Ammoniumsulphat in einer Konzentration von 5 % enthält, wurde einer Umkehrosmose und einer Eindampfung unterzogen. Nach der Umkehrosmose beträgt die Konzentration des Ammoniumsulfats im Raffinat 12 %. Diese Lösung wurde dann einer weiteren Aufkonzentrierung auf 40 % durch eine Eindampfung unterzogen. Die aufkonzentrierte Lösung enthielt einen Gehalt an Ammoniumsulphat von 40 %. Zur Eindampfung wurden 2,1 t Dampf pro t Ammoniumsulfat eingespeist. Aus der Eindampfungseinheit austretender Dampf wurde zur Eindampfungseinheit zur Aufkonzentrierung des Bernsteinsäure enthaltenden Extraktes geleitet (ca. 2,1 t Dampf pro t Bernsteinsäure) und dort weiter benutzt. Durch die Kopplung mit einer Umkehrosmose musste kein zusätzlicher Frischdampf der Eindampfungseinheit der Carbonsäure zugeführt werden.

Vorteile des erfindungsgemäßen Verfahrens:
- durch die erfindungsgemäßen Kombination aus Umkehrosmose und Eindampfung und die zusätzliche Weiterverarbeitung des Dampfes aus der Raffinatkonzentration aus Verfahrensschritt f) sind erhebliche Einsparungen an Dampf und somit Energie möglich.
- das Verfahren der Aufreinigung von Carbonsäuren, ausgewählt aus der Gruppe umfassend Hydroxycarbonsäuren und Dicarbonsäuren, aus Fermentationsbrühen wird erheblich wirtschaftlicher gestaltet, als Verfahren aus dem Stand der Technik

## Patentansprüche

1. Verfahren zur Abtrennung und Aufreinigung von aus der Gruppe umfassend Hydroxycarbonsäuren und Dicarbonsäuren ausgewählten Carbonsäuren aus Fermentationsbrühen enthaltend Ammoniumcarbonsäuresalze, wobei das Verfahren die folgenden Schritte umfasst,
a) eine Abtrennung von Biomasse und eventuell vorhandener Feststoffe aus der Fermentationsbrühe,
b) Herstellung einer Lösung enthaltend die gewünschte Carbonsäure und einer weiteren Lösung enthaltend Ammoniumsalze, indem die biomassefreie Fermentationsbrühe mit konzentrierter Schwefelsäure angesäuert und einer Simulated Moving Bed Chromatographie (SMB) unterzogen wird ,
c) eine Feinreinigung der Lösung enthaltend die gewünschte Carbonsäure aus Verfahrensschritt b),
d) eine Aufkonzentrierung der aufgereinigten Carbonsäurelösung aus Verfahrensschritt c),
e) Kristallisation der aufkonzentrierten Carbonsäurelösung aus Verfahrensschritt d)
f) eine Aufkonzentrierung der weiteren Lösung enthaltend Ammoniumsalze aus Verfahrensschritt b),
**dadurch gekennzeichnet, dass**
in Verfahrensschritt d) und f) bei der Aufkonzentrierung eine Kombination aus Umkehrosmose und Eindampfung durchgeführt wird und der Dampf aus der Eindampfung des Verfahrensschrittes f) in die Eindampfung aus Verfahrensschritt d) geleitet wird.

2. Verfahren nach Anspruch 1, **dadurch gekennzeichnet, dass** Verfahrensschritt f) derart ausgelegt wird, dass in Verfahrensschritt d) im Normalbetrieb keinerlei Frischdampf zuzuführen ist oder 1 bis 10 t Frischdampf pro t Produktcarbonsäure zuzuführen ist, besonders bevorzugt 1,5 bis 4 t Frischdampf pro t Produktcarbonsäure zuzuführen ist.

3. Verfahren nach einem der vorstehenden Ansprüche, **dadurch gekennzeichnet, dass** entstehendes Permeat aus der Umkehrosmose als Eluent in Verfahrensschritt b) eingesetzt wird.

4. Verfahren nach einem der vorstehenden Ansprüche, **dadurch gekennzeichnet, dass** die Abtrennung der Biomasse aus der Fermentationsbrühe in Verfahrensschritt a) durch eine Precoat- und/oder eine Mikrofiltration und/oder eine Ultrafiltration erfolgt und die abgetrennte Biomasse in Verfahrensschritt a) wieder in den Fermenter rückgeführt wird.

5. Verfahren nach einem der vorstehenden Ansprüche, **dadurch gekennzeichnet, dass** die Abtrennung der Biomasse aus der Fermentationsbrühe in Verfahrensschritt a) ohne Absenken des pH-Wertes und ohne thermische Inaktivierung erfolgt.

6. Verfahren nach einem der vorstehenden Ansprüche, **dadurch gekennzeichnet, dass** in Verfahrensschritt a) die Zeit zwischen Beendigung der Fermentation und der Abtrennung der Biomasse so kurz als möglich gehalten wird und diese Zeit vorzugsweise nicht mehr als 2h beträgt, und besonders bevorzugt weniger als 1 - 2h beträgt.

7. Verfahren nach einem der vorstehenden Ansprüche, **dadurch gekennzeichnet, dass** in Verfahrensschritt a) die Biomassekonzentration im Filtrat nicht höher als 1 g/l ist.

8. Verfahren nach einem der vorstehenden Ansprüche, **dadurch gekennzeichnet, dass** das Permeat aus der Filtration aus Verfahrensschritt a) mit konzentrierter Schwefelsäure auf einen pH-Wert von 2,2 bis 2,4 angesäuert wird, wobei die Temperatur des angesäuerten Permeates der Ultrafiltration in einem Bereich zwischen 30°C und 60°C, und vorzugsweise in einem Bereich zwischen 30°C und 40°C gehalten wird.

9. Verfahren nach einem der vorstehenden Ansprüche, **dadurch gekennzeichnet, dass** in Verfahrensschritt b)
- die Zugabe des Permeates aus der Filtration aus Verfahrensschritt a) und eines Eluents kontinuierlich in einem Verhältnis Permeat : Eluent von 1 : 1,5 bis 1 : 2,5 erfolgt,
- die Carbonsäure an eine stationäre Phase der SMB bindet, wobei diese aus einem Kationenaustauscher und/oder einem Anionenaustauscher aufgebaut ist,
- der die Carbonsäure enthaltende Extrakt und das Raffinat, aufweisend einen Gehalt an Dicarbonsäure ≤ 1 g/l, getrennt voneinander gesammelt werden,
- der Wirkungsgrad der Gewinnung von Carbonsäure aus dem Permeat der Filtration aus Verfahrensschritt a) ≥ 95% beträgt.

10. Verfahren nach einem der vorstehenden Ansprüche, **dadurch gekennzeichnet, dass** das Extrakt aus der SMB-Chromatographie aus Verfahrensschritt b) in Verfahrensschritt c) einer Nanofiltration unterzogen wird, wobei die Membranen eine Trenngrenze von 100 bis 400 Da, vorzugsweise 200 Da, besitzen und/oder in Verfahrensschritt c) eine Feinreinigung durch Aktivkohlefiltration und/oder Kationenaustauscher und/oder Anionenaustauscher durchgeführt wird.

11. Verfahren nach einem der vorstehenden Ansprüche, **dadurch gekennzeichnet, dass** in Verfahrensschritt d) mindestens einer oder mehrere Verfahrensschritte zur Umkehrosmose mit mindestens einem oder mehreren Verfahrensschritten zur Eindampfung kombiniert werden.

12. Verfahren nach einem der vorstehenden Ansprüche, **dadurch gekennzeichnet, dass** die abzutrennenden und aufzureinigenden Carbonsäuren ausgewählt werden aus der Gruppe umfassend Hydroxycarbonsäuren und Dicarbonsäuren, und bevorzugt ausgewählt werden aus der Gruppe umfassend Äpfelsäure, Glycolsäure, Isozitronensäure, Mandelsäure, Milchsäure, Tartronsäure, Weinsäure, Zitronensäure, ß-Hydroxybuttersäure, Mevalonsäure, Salicylsäure, Oxalsäure, Maleinsäure, Bernsteinsäure, Glutarsäure, Adipinsäure, Pimelinsäure, Korksäure, Fumarsäure und Itaconsäure.

13. Verwendung einer Vorrichtung zur Durchführung eines Verfahrens nach Anspruch 1 umfassend
a) eine oder mehrere Filtrationseinrichtungen zur Abtrennung der Biomasse und eventuell vorhandenen Feststoffen aus der Fermentationsbrühe,
b) einen Verfahrensschritt zur Simulated Moving Bed Chromatographie (SMB) nach Ansäuerung, wobei eine Lösung enthaltend die gewünschte Carbonsäure und eine weitere Lösung enthaltend Ammoniumsalze entsteht,
c) einen Verfahrensschritt zur Feinreinigung der Lösung enthaltend die gewünschte Carbonsäure aus ein Verfahrensschritt b)
d) einen Verfahrensschritt zur Aufkonzentrierung der aufgereinigten Carbonsäurelösung aus ein Verfahrensschritt c)
e) einen Verfahrensschritt zur Kristallisation der aufkonzentrierten Carbonsäurelösung aus Verfahrensschritt d)
f) einen Verfahrensschritt zur Aufkonzentrierung der weiteren Lösung enthaltend Ammoniumsalze aus Verfahrensschritt b)
**dadurch gekennzeichnet, dass**
Verfahrensschritt d) und f) eine Kombination aus einer oder mehreren Vorrichtungen zur Umkehrosmose und einer oder mehreren Vorrichtungen zur Eindampfung aufweist, und Überleitungen vorhanden sind, um den Dampf aus der Eindampfung aus Verfahrensschritt f) in die Eindampfung aus Verfahrensschritt d) zu überführen.

14. Verwendung einer Vorrichtung nach Anspruch 13, **dadurch gekennzeichnet, dass** in Verfahrensschritt d) Anlagenteile zur Generierung von Frischdampf auf eine Frischdampfgenerierung von 1 bis 10 t Frischdampf pro t Produktcarbonsäure ausgelegt sind, und insbesondere auf eine Frischdampfgenerierung von 1,5 bis 4 t Frischdampf pro t Produktcarbonsäure ausgelegt sind.

15. Verwendung einer Vorrichtung nach Anspruch 14, **dadurch gekennzeichnet, dass** Überleitungen vorhanden sind, um Permeat aus der Umkehrosmose in die Simulated Moving Bed Chromatographie aus Verfahrensschritt b) zu überführen.

## Claims

1. Method for separating and purifying carboxylic acids selected from the group comprising hydroxycarboxylic acids and dicarboxylic acids from fermentation broths comprising carboxylic acid ammonium salts, wherein the method comprises the following steps,
a) removing biomass and any solids present from the fermentation broth,
b) preparing a solution comprising the desired carboxylic acid and an additional solution comprising ammonium salts, by acidifying the biomass-free fermentation broth with concentrated sulfuric acid and subjecting it to simulated moving bed chromatography (SMB),
c) ultra-purifying the solution comprising the desired carboxylic acid from method step b),
d) concentrating the purified carboxylic acid solution from method step c),
e) crystallizing the concentrated carboxylic acid solution from method step d)
f) concentrating the additional solution comprising ammonium salts from method step b),
**characterized in that,**
in the concentration in method steps d) and f), a combination of reverse osmosis and evaporation is carried out and the vapor from the evaporation of method step f) is passed into the evaporation of method step d).

2. Method according to Claim 1, **characterized in that** method step f) is designed such that in method step d) under normal operation no fresh vapor is to be supplied or 1 to 10 t of fresh vapor per t of carboxylic acid product is to be supplied, particularly preferably 1.5 to 4 t of fresh vapor per t of carboxylic acid product is to be supplied.

3. Method according to any of the preceding claims, **characterized in that** the permeate resulting from the reverse osmosis is used as eluent in method step b).

4. Method according to any of the preceding claims, **characterized in that** the removal of the biomass from the fermentation broth in method step a) is effected by precoat filtration and/or microfiltration and/or ultrafiltration and the biomass removed in method step a) is fed back again into the fermenter.

5. Method according to any of the preceding claims, **characterized in that** the removal of the biomass from the fermentation broth in method step a) is carried out without lowering the pH and without thermal inactivation.

6. Method according to any of the preceding claims, **characterized in that** in method step a) the time between completion of the fermentation and the removal of the biomass is kept as short as possible, and this time is preferably not more than 2 h and particularly preferably less than 1-2 h.

7. Method according to any of the preceding claims, **characterized in that** in method step a) the biomass concentration in the filtrate is not higher than 1 g/l.

8. Method according to any of the preceding claims, **characterized in that** the permeate from the filtration of method step a) is acidified with concentrated sulfuric acid to a pH of 2.2 to 2.4, wherein the temperature of the acidified permeate of the ultrafiltration is maintained in a range between 30°C and 60°C, and preferably in a range between 30°C and 40°C.

9. Method according to any of the preceding claims, **characterized in that** in method step b)
- the addition of the permeate from the filtration from method step a) and of an eluent is carried out continuously in a ratio of permeate : eluent of 1 : 1.5 to 1 : 2.5,
- the carboxylic acid binds to a stationary phase of the SMB, wherein said stationary phase is composed of a cation exchanger and/or an anion exchanger,
- the extract comprising the carboxylic acid and the raffinate, having a dicarboxylic acid content of ≤ 1 g/l, are collected separately from each other,
- the efficiency of the recovery of carboxylic acid from the permeate of the filtration from method step a) is ≥ 95%.

10. Method according to any of the preceding claims, **characterized in that** the extract from the SMB chromatography from method step b) is subjected to a nanofiltration in method step c), wherein the membranes have a cut-off of 100 to 400 Da, preferably 200 Da, and/or an ultra-purification by activated carbon filtration and/or cation exchange and/or anion exchange is carried out in method step c).

11. Method according to any of the preceding claims, **characterized in that** at least one or more method steps for the reverse osmosis are combined with at least one or more method steps for the evaporation in method step d).

12. Method according to any of the preceding claims, **characterized in that** the carboxylic acids to be separated and purified are selected from the group comprising hydroxycarboxylic acids and dicarboxylic acids, and are preferably selected from the group comprising malic acid, glycolic acid, isocitric acid, mandelic acid, lactic acid, tartronic acid, tartaric acid, citric acid, β-hydroxybutyric acid, mevalonic acid, salicylic acid, oxalic acid, maleic acid, succinic acid, glutaric acid, adipic acid, pimelic acid, suberic acid, fumaric acid and itaconic acid.

13. Use of a device for carrying out a method according to Claim 1 comprising
a) one or more filtration devices for removing biomass and any solids present from the fermentation broth,
b) a method step for simulated moving bed chromatography (SMB) after acidification, wherein a solution comprising the desired carboxylic acid and an additional solution comprising ammonium salts is produced,
c) a method step for ultra-purifying the solution comprising the desired carboxylic acid from method step b)
d) a method step for concentrating the purified carboxylic acid solution from method step c)
e) a method step for crystallizing the concentrated carboxylic acid solution from method step d)
f) a method step for concentrating the additional solution comprising ammonium salts from method step b),
**characterized in that,**
method steps d) and f) comprise a combination of one or more devices for reverse osmosis and one or more devices for evaporation, and connections are in place to transfer the vapor from the evaporation of method step f) to the evaporation of method step d).

14. Use of a device according to Claim 13, **characterized in that** in method step d) plant components are designed for generating fresh vapor with a fresh vapor generation of 1 to 10 t of fresh vapor per t of carboxylic acid product, and are particularly designed for a fresh vapor generation of 1.5 to 4 t of fresh vapor per t of carboxylic acid product.

15. Use of a device according to Claim 14, **characterized in that** connections are in place to transfer the permeate from the reverse osmosis into the simulated moving bed chromatography of method step b).

## Revendications

1. Procédé pour la séparation et la purification d'acides carboxyliques choisis dans le groupe comprenant les acides hydroxycarboxyliques et les acides dicarboxyliques à partir de bouillons de fermentation contenant des sels d'ammonium d'acides carboxyliques, le procédé comprenant les étapes suivantes :
a) séparation de biomasse et de solides éventuellement présents du bouillon de fermentation,
b) préparation d'une solution contenant l'acide carboxylique souhaité et d'une autre solution contenant des sels d'ammonium en ce que le bouillon de fermentation exempt de biomasse est acidifié par de l'acide sulfurique concentré et soumis à une chromatographie à lit mobile simulé (Simulated Moving Bed - SMB),
c) purification fine de la solution contenant l'acide carboxylique souhaité provenant de l'étape de procédé b),
d) concentration de la solution d'acide carboxylique purifiée provenant de l'étape de procédé c),
e) cristallisation de la solution d'acide carboxylique concentrée provenant de l'étape de procédé d),
f) concentration de l'autre solution contenant des sels d'ammonium provenant de l'étape de procédé b),
**caractérisé en ce que**, dans les étapes de procédé d) et f), lors de la concentration, une combinaison d'une osmose inverse et d'une évaporation est réalisée et la vapeur provenant de l'évaporation de l'étape de procédé f) est guidée dans l'évaporation de l'étape de procédé d).

2. Procédé selon la revendication 1, **caractérisé en ce que** l'étape de procédé f) est conçue de manière telle que dans l'étape de d), lors du fonctionnement normal, aucune vapeur fraîche ne doit être introduite ou 1 à 10 t de vapeur fraîche par t d'acide carboxylique produite doit être introduite, de manière particulièrement préférée 1,5 à 4 t de vapeur fraîche par t d'acide carboxylique produite.

3. Procédé selon l'une quelconque des revendications précédentes, **caractérisé en ce que** le perméat formé provenant de l'osmose inverse est utilisé comme éluent dans l'étape de procédé b).

4. Procédé selon l'une quelconque des revendications précédentes, **caractérisé en ce que** la séparation de la biomasse à partir du bouillon de fermentation dans l'étape de procédé a) a lieu par une filtration à précouche et/ou une microfiltration et/ou une ultrafiltration et la biomasse séparée dans l'étape de procédé a) est de nouveau recyclée dans le fermenteur.

5. Procédé selon l'une quelconque des revendications précédentes, **caractérisé en ce que** la séparation de la biomasse du bouillon de fermentation dans l'étape de procédé a) a lieu sans diminution du pH ni inactivation thermique.

6. Procédé selon l'une quelconque des revendications précédentes, **caractérisé en ce que**, dans l'étape de procédé a), le temps entre la fin de la fermentation et la séparation de la biomasse est maintenu le plus court possible et ce temps n'est de préférence pas supérieur à 2 h et de manière particulièrement préférée inférieur à 1-2 h.

7. Procédé selon l'une quelconque des revendications précédentes, **caractérisé en ce que**, dans l'étape de procédé a), la concentration en biomasse dans le filtrat n'est pas supérieure à 1 g/l.

8. Procédé selon l'une quelconque des revendications précédentes, **caractérisé en ce que** le perméat de la filtration de l'étape de procédé a) est acidifié avec de l'acide sulfurique concentré à une valeur de pH de 2,2 à 2,4, la température du perméat acidifié de l'ultrafiltration étant maintenue dans une plage entre 30°C à 60°C et de préférence dans une plage entre 30°C à 40°C.

9. Procédé selon l'une quelconque des revendications précédentes, **caractérisé en ce que**, dans l'étape de procédé b),
- l'addition du perméat provenant de la filtration de l'étape de procédé a) et d'un éluant a lieu en continu dans un rapport perméat:éluant de 1:1,5 à 1:2,5,
- l'acide carboxylique se lie à une phase stationnaire de la chromatographie SMB, celle-ci étant constituée par un échangeur cationique et/ou un échangeur anionique,
- l'extrait contenant l'acide carboxylique et le raffinat, présentant une teneur en acide dicarboxylique ≤ 1 g/l, sont récupérés séparément l'un de l'autre,
- le degré d'efficacité de la production d'acide carboxylique à partir du perméat de la filtration de l'étape de procédé a) est ≥ 95%.

10. Procédé selon l'une quelconque des revendications précédentes, **caractérisé en ce que** l'extrait provenant de la chromatographie SMB de l'étape de procédé b) est soumis à une nanofiltration dans l'étape de procédé c), les membranes présentant une limite de séparation de 100 à 400 Da, de préférence de 200 Da, et/ou, dans l'étape de procédé c), un réglage fin par filtration sur charbon actif et/ou sur un échangeur cationique et/ou sur un échangeur anionique est réalisé.

11. Procédé selon l'une quelconque des revendications précédentes, **caractérisé en ce que**, dans l'étape de procédé d), au moins une ou plusieurs étapes de procédé pour l'osmose inverse sont combinées avec au moins une ou plusieurs étapes de procédé pour l'évaporation.

12. Procédé selon l'une quelconque des revendications précédentes, **caractérisé en ce que** les acides carboxyliques à séparer et à purifier sont choisis dans le groupe comprenant les acides hydroxycarboxyliques et les acides dicarboxyliques et sont de préférence choisis dans le groupe comprenant l'acide malique, l'acide glycolique, l'acide isocitrique, l'acide mandélique, l'acide lactique, l'acide tartronique, l'acide tartrique, l'acide citrique, l'acide β-hydroxybutyrique, l'acide mévalonique, l'acide salicylique, l'acide oxalique, l'acide maléique, l'acide succinique, l'acide glutarique, l'acide adipique, l'acide pimélique, l'acide subérique, l'acide fumarique et l'acide itaconique.

13. Utilisation d'un dispositif pour réaliser un procédé selon la revendication 1, comprenant
a) un ou plusieurs dispositifs de filtration pour la séparation de la biomasse et des solides éventuellement présents à partir du bouillon de fermentation,
b) une étape de procédé pour la chromatographie à lit mobile simulé (Simulated Moving Bed - SMB) après acidification, une solution contenant l'acide carboxylique souhaité et une autre solution contenant des sels d'ammonium étant formées,
c) une étape de procédé pour la purification fine de la solution contenant l'acide carboxylique souhaité provenant de l'étape de procédé b),
d) une étape de procédé pour la concentration de la solution d'acide carboxylique purifiée provenant de l'étape de procédé c),
e) une étape de procédé pour la cristallisation de la solution d'acide carboxylique concentrée provenant de l'étape de procédé d),
f) une étape de procédé pour la concentration de l'autre solution contenant des sels d'ammonium provenant de l'étape de procédé b),
**caractérisée en ce que** les étapes de procédé d) et f) présentent une combinaison d'un ou de plusieurs dispositifs pour l'osmose inverse et d'un ou de plusieurs dispositifs pour l'évaporation et des dérivations sont présentes pour transférer la vapeur provenant de l'évaporation de l'étape de procédé f) dans l'évaporation de l'étape de procédé d).

14. Utilisation d'un dispositif selon la revendication 13, **caractérisée en ce que**, dans l'étape de procédé d), des pièces d'installations pour générer de la vapeur fraîche sont conçues pour une génération de vapeur fraîche de 1 à 10 t de vapeur fraîche par t d'acide carboxylique produite et en particulier pour une génération de vapeur fraîche de 1,5 à 4 t de vapeur fraîche par t d'acide carboxylique produite.

15. Utilisation d'un dispositif selon la revendication 14, **caractérisée en ce que** des dérivations sont présentes pour transférer le perméat provenant de l'osmose inverse dans la chromatographie à lit mobile simulé de l'étape de procédé b).
